(19) Europäisches Patentamt
European Patent Office
Office européen des brevets

(11) Veröffentlichungsnummer: **0 071 899**
**B1**

(12) # EUROPÄISCHE PATENTSCHRIFT

(45) Veröffentlichungstag der Patentschrift: **20.03.85**

(51) Int. Cl.[4]: **C 07 D 229/00**, C 08 G 18/79

(21) Anmeldenummer: **82106878.0**

(22) Anmeldetag: **30.07.82**

(54) **Verfahren zur Herstellung von niedermolekularen 4,4'-Diphenylmethan-uretdion-diisocyanaten.**

(30) Priorität: **12.08.81 DE 3131779**

(43) Veröffentlichungstag der Anmeldung:
**16.02.83 Patentblatt 83/7**

(45) Bekanntmachung des Hinweises auf die Patenterteilung:
**20.03.85 Patentblatt 85/12**

(84) Benannte Vertragsstaaten:
**BE DE FR GB IT NL**

(56) Entgegenhaltungen:
**DE-A-1 445 721**
**FR-A-2 268 838**
**GB-A-1 207 673**

(73) Patentinhaber: **BAYER AG, Konzernverwaltung RP Patentabteilung, D-5090 Leverkusen 1 Bayerwerk (DE)**

(72) Erfinder: **Grögler, Gerhard, Dr., von-Diergardt-Strasse 46, D-5090 Leverkusen 1 (DE)**
Erfinder: **Kopp, Richard, Dr., Wolfskaul 12, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 899 B1

## Beschreibung

Die Erfindung betrifft ein Verfahren zur Herstellung von niedermolekularen 4,4'-Diphenylmethanuretdiondiisocyanaten der Formel

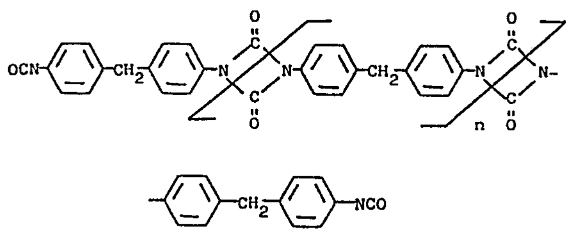

wobei n 0 bis 1, vorzugsweise 0 bis 0,5, beudeutet, bei dem man Diphenylmethan-4,4'-diisocyanat unter schonenden Dimerisierungsbedingungen in einem Gemisch von mässig polaren und weitgehend unpolaren Lösungsmitteln dimerisiert und bei relativ niedrigen Temperaturen schonend aufarbeitet.

Aromatische Uretdiondiisocyanate (dimere Diisocyanate) sowie deren Herstellungsverfahren sind bekannt. Vergleiche „Kunststoff-Handbuch", Band VII, Polyurethane, herausgegeben von Vieweg-Höchtlen, Carl-Hanser-Verlag München, 1966.

Als Dimerisierungskatalysatoren wurden neben Trialkylphosphinen („J. Org. Chem.", *8*, 23 [1943]), aromatischaliphatische tertiäre Phosphine, Alkyldiarylphosphine (DE-A Nr. 2452390), 3- oder 4-substituierte Pyridine (GB-PS Nr. 821158), Trialkylphosphite (DE-A Nr. 2349726) sowie Phosphorigsäuretrisdialkylamide (US-PS Nr. 3290288) genannt. Die Dimerisierungsreaktion zum Uretdion kann dabei in Einzelfällen sogar in Substanz erfolgen. Vorwiegend werden aber Lösungsmittel verwendet, die sich gegenüber NCO-Gruppen inert verhalten. Als Lösungsmittel werden z.B. genannt: Benzol, Toluol, Xylol, Chlorbenzol, Nitrobenzol, Aceton, Methylethylketon, Essigester, Dioxan, Tetrahydrofuran, aliphatische Kohlenwasserstoffe, Dimethylformamid und Methylenchlorid. Eine Differenzierung der genannten Lösungsmittel hinsichtlich ihrer Polarität bzw. ihrem Lösungsvermögen gegenüber den Anfangs- bzw. Endprodukten der Reaktion wird in der Patentliteratur nicht vorgenommen.

Dass bei der Dimerisierung von aromatischen Isocyanaten, insbesondere bei reaktiven Dimerisierungskatalysatoren, unerwünschte Nebenprodukte, wie z.B. Isocyanurat, entstehen können, wird bereits in der US-PS Nr. 2683144 beschrieben. Es wird daher empfohlen, die Dimerisierung dann zu unterbrechen, wenn eine gewünschte Dimerisierungsstufe erreicht ist. Als Inhibitoren (Stopper) für den Dimerisierungskatalysator werden z.B. Alkylierungsmittel insbesondere Cyclohexansulfonsäuremethylester oder Benzylchlorid verwendet. Aromatische Diisocyanate, bei denen eine NCO-Gruppe in einer oder beiden Orthostellungen durch andere Reste R substituiert sind, z.B. Alkylgruppen, ergeben nach der vorliegenden Literatur Uretdiondiisocyanate mit dem doppelten Molgewicht der Ausgangsverbindungen, z.B. das dimere Toluylendiisocyanat

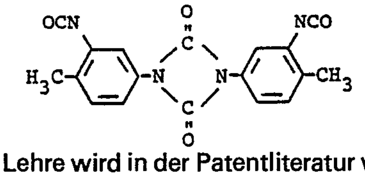

Diese Lehre wird in der Patentliteratur weiterhin übertragen auf solche aromatische Diisocyanate, bei denen die aromatisch gebundene NCO-Gruppe in Orthostellung lediglich H-Atome aufweist. Die vielfach in der Patentliteratur übliche Bezeichnung für solche Uretdiondiisocyanate, z.B. auf Basis von 4,4'-Diphenylmethandiisocyanat, lautet daher Diphenylmethandiisocyanatdimer und wird folgerichtig durch die Formel

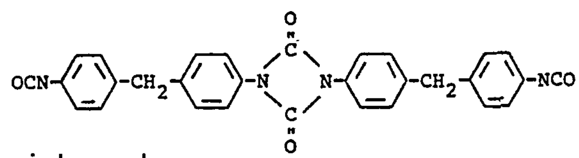

wiedergegeben.

Erstmalig wurden in der DE-A Nr. 1445721 (US-PS Nr. 3290288) differenzierte Angaben über oligomere Uretdiondiisocyanatderivate auf Basis von 4,4'-Diphenylmethandiisocyanat gebracht, wobei erwähnt wird, dass bei der Dimerisierung von Diphenylmethandiisocyanat als Endprodukte neben di-, auch tri-, tetra- und pentamere Uretdione erhalten werden

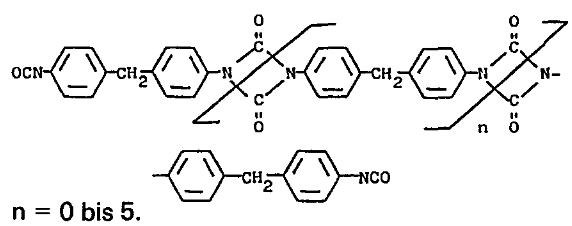

n = 0 bis 5.

Die Verwendung von dimerisiertem Diphenylmethandiisocyanat nach obiger Literatur wird in der DE-A Nr. 2419968 zum Aufbau von hochmolekularen Polyurethanen mit Uretdiongruppen durch Reaktion in hochpolaren Lösungsmitteln wie DMF beschrieben.

Mit zunehmendem Polydimerisierungsgrad (n etwa grösser als 2,5) nimmt aber infolge erhöhter Schwerlöslichkeit dieser Uretdiondiisocyanate die Reaktivität gegenüber H-aciden Verbindungen deutlich ab, so dass solche höhermolekularen Oligomere für viele Polyurethanreaktionen, besonders wenn sie lösungsmittelfrei bzw. bei Temperaturen unterhalb der Schmelzpunkte der Uretdiondiisocyanate durchgeführt werden, praktisch nicht mehr geeignet sind.

In der Patentliteratur wird nur in wenigen Fällen auf die Bedeutung des Lösungsmittels bei der Dimerisierungsreaktion eingegangen. Nicht erwähnt bleibt der Einfluss des Lösungsmittels, insbesondere bei der Dimerisierung von aromatischen Diisocyanaten, bei denen die NCO-Gruppe in Orthostellungen nur von H-Atomen flankiert ist. Einige in der Patentliteratur zur Dimerisierung von aromatischen Isocyanaten als geeignet angegebene inerte Lösungsmittel erweisen sich bei der Dimerisierung von Diphenylmethandiisocyanat als unbrauchbar, da hierbei hochmolekulare MDI-Dimergemische (n sehr viel grösser als 2,5) entstehen, die für weitere Polyurethanreaktionen zumeist nicht mehr geeignet sind (vgl. Beispiel 1). So beschreibt die GB-PS Nr. 1207673 die Herstellung von Linearpolymeren des 4,4'-Diisocyanatdiphenylmethans mit Pyridin- oder Phosphinkatalysatoren in NCO-inerten Lösungsmitteln bei Temperaturen von höchstens 50°C.

In sehr hochpolaren Lösungsmitteln, wie Dimethylformamid oder Dimethylacetamid entstehen praktisch ausschliesslich Polydimere, die sich in ihrer Reaktivität und Umsetzbarkeit deutlich von den erfindungsgemäss zugänglichen Verbindungen unterscheiden.

Der Erfindung lag daher die Aufgabe zugrunde, Uretdiondiisocyanate auf Basis Diphenylmethan-4,4'-diisocyanat mit möglichst geringem Oligomerisierungsgrad ($n \leqslant 1$, vorzugsweise $\leqslant 0,5$) herzustellen, welche sich noch gut in den Polyadditionsreaktionen umsetzen lassen.

Es wurde nun überraschend gefunden, dass nicht die Wahl oder die Menge des Katalysators innerhalb der üblichen Grenzen oder das kontrollierte Abstoppen des Katalysators bei einem bestimmten Dimerisierungsgrad für die selektive Bildung von möglichst niedermolekularen Diphenylmethanuretdiondiisocyanaten verantwortlich ist, sondern es konnte vielmehr nachgewiesen werden, dass die Wahl des Lösungsmittels und eine möglichst niedrige Dimerisierungstemperatur sowie insbesondere eine möglichst niedrige Aufarbeitungstemperatur bei der Isolierung der Dimeren von ausschlaggebender Bedeutung ist.

So werden bei der Dimerisierung von Diphenylmethan-4,4'-diisocyanat in einem Gemisch von unpolaren und polaren Lösungsmitteln Diphenylmethanuretdiondiisocyanate mit sehr geringem Polyadditionsgrad n erhalten ($n \leqslant 1$, vorzugsweise $\leqslant 0,5$), insbesondere dann, wenn das unpolare Lösungsmittel nur ein geringes Lösungsvermögen für Diphenylmethan-4,4'-diisocyanat (5 bis 25, vorzugsweise 5 bis 15 Gew.-%) aufweist. Wird die Dimerisierung von Diphenylmethandiisocyanat in solch einem unpolaren Lösungsmittel allein durchgeführt, so erhält man zwar auch niedermolekulare Diphenylmethanuretdiondiisocyanate, dies Verfahren ist aber infolge der grossen Verdünnung wenig wirtschaftlich und darüberhinaus enthalten solche Diphenylmethanuretdiondiisocyanate noch relativ viel eingeschlossene monomere Diphenylmethandiisocyanatstartkomponente.

Durch Zugabe eines geeigneten, mässig polaren NCO-inerten Lösungsmittels kann die Löslichkeit von Diphenylmethandiisocyanat im Lösungsmittelgemisch deutlich erhöht werden, ohne die gezielte Dimerisierung zu niedermolekularem Diphenylmethanuretdiondiisocyanat zu beeinflussen. Hierbei fällt das primär gebildete Diphenylmethanuretdiondiisocyanat ($n \geqslant 0$) aus und oligomerisiert nicht merklich weiter.

Ein weiterer entscheidender Vorteil ist bei dieser Arbeitsweise, dass das fast einheitliche Diphenylmethanuretdiondiisocyanat ($n \leqslant 1$, vorzugsweise $\leqslant 0,5$) in sehr hohen Ausbeuten (90 bis 98% der Theorie) erhalten wird.

Gegenstand der Erfindung ist somit ein verbessertes Verfahren zur Herstellung von niedermolekularen 4,4-Diphenylmethanuretdiondiisocyanaten der Formel

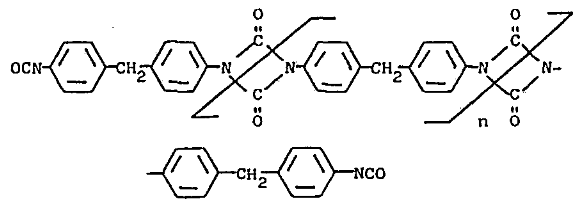

wobei n 0 bis 1, vorzugsweise 0 bis 0,5 bedeutet, durch Dimerisierung von Diphenylmethan-4,4'-diisocyanat in NCO-inerten Lösungsmitteln in Gegenwart von Dimerisierungskatalysatoren und gegebenenfalls Abstoppern, dadurch gekennzeichnet, dass man die Dimerisierung in Gegenwart von Dimerisierungskatalysatoren in einem Gemisch aus unpolaren Lösungsmitteln und mässig polaren Lösungsmitteln durchführt, wobei die Löslichkeit des Diphenylmethan-4,4'-diisocyanats in den unpolaren Lösungsmitteln 5 bis 25, vorzugsweise 5 bis 15 Gew.-%, beträgt, und dass man während der Dimerisierungsreaktion und insbesondere bei der Aufarbeitung der Uretdiondiisocyanate Temperaturen unter 50, vorzugsweise unterhalb von 30°C, einhält.

Als Dimerisierungskatalysatoren werden die üblichen Katalysatoren, wie sie bereits aufgeführt waren, eingesetzt, insbesondere jedoch Trialkylphosphine, wie Triethylphosphin oder Tributylphosphin. Die Katalysatoren werden in üblichen Mengen, z.B. 0,05 bis 5, vorzugsweise jedoch 0,1 bis 1,0 und besonders bevorzugt bei 0,1 bis 0,35 Gew.-%, eingesetzt.

Als unpolare Lösungsmittel sind aliphatische und/oder cycloaliphatische Kohlenwasserstoffe, wie Hexan, Octan, Cyclopentan, Cyclohexan, Cyclododecan, sowie entsprechende Kohlenwasserstoffdestillate mit überwiegendem Gehalt an aliphatischen und/oder cycloaliphatischen Kohlenwasserstoffen, z.B. Petrolether, Waschbenzin, Ligroin, geeignet. Die Dielektrizitätskonstante derartiger Lösungsmittel liegt im allgemeinen unter 2,10. Besonders bevorzugt sind Kohlenwasserstoffdestillate wie Petrolether, Ligroin und Leichtbenzin. Als mässig polare Lösungsmittel sind z.B. aromatische Kohlenwasserstoffe, gegebenenfalls ihre chlorierten Derivate, Carbonsäureester, Ketone und Ether geeignet. Ihre Dielektrizitätskonstanten betragen etwa 2,24 bis 10, können im Falle von Keton jedoch höher (bis ca. 22) liegen. Beispiele sind Benzol, Toluol, Xylol, Chlorbenzol, Essigsäuremethylester, Essigsäureethylester, Propionsäurediethylester, Ameisensäurediethylester, Phthalsäurediethylester, Phthalsäuredioctylester, Aceton, Methylethylketon, Cyclohexanon, Isophoron, Diethylether, Tetrahydrofuran und Tetrachlorkohlenstoff. Aromatische Kohlenwasserstoffe, insbesondere Toluol, sind bevorzugt.

Das Verhältnis von unpolaren zu mässig polaren Lösungsmitteln ergibt sich aus der Löslichkeit von

Diphenylmethandiisocyanat in diesem Lösungsmittelgemisch. Die Lösungsmittelgemische liegen im allgemeinen zwischen 2:1 bis 1:2, vorzugsweise wird bei einem Verhältnis von etwa 1:1 gearbeitet.

Man kann aber auch so verfahren, dass 1 bis 2 Teile an unpolarem Lösungsmittel bei Raumtemperatur mit 1 Teil Diphenylmethandiisocyanat bis zur Sättigung gerührt werden und anschliessend soviel polares Lösungsmittel zugesetzt wird, bis eine homogene Lösung entsteht. Die Konzentration von Diphenylmethandiisocyanat in diesem Lösungsmittelgemisch beträgt dann im allgemeinen 25 bis 60, vorzugsweise 30 bis 50 Gew.-%.

Man kann auch hochkonzentrierte Lösungen von Diphenylmethandiisocyanat in den mässig polaren Lösungsmitteln mit unpolaren Lösungsmitteln versetzen.

Die weiteren Dimerisierungsbedingungen wie Temperaturführung, Wahl und Menge des Katalysators, Reaktionszeiten, Abstoppen der Katalysatoren wurden in der vorliegenden Literatur hinreichend beschrieben. Es ist jedoch wichtig, bei der Dimerisierungsreaktion die Reaktionstemperaturen nicht so hoch ansteigen zu lassen (Temperatur $\leqslant$ 50, vorzugsweise $\leqslant$ 30°C). Ferner ist es sehr empfehlenswert, den Katalysator nach Erreichen der gewünschten Di- und/oder Oligomerisierungsschritte in bekannter Weise abzustoppen.

Ferner darf in der Aufarbeitungsstufe des Dimeren eine nur schonende Temperaturbehandlung erfolgen, da sonst eine weitere Uretdionbildungsreaktion aus den freien NCO-Gruppen der Dimeren erfolgt und sich dann polymere Uretdione bilden. Letztere sind ausserordentlich schwer löslich und enthalten wenig freie NCO-Gruppen und lassen sich ausserordentlich schwer weiter umsetzen. Daher dürfte auch die bisher ausstehende Nutzung sogenannter Dimeren des Diphenylmethan-4,4'-diisocyanats resultieren, da sie nach bisherigen Methoden in polymerer oder hocholigomerisierter (und nicht dimerer) Form erhalten wurden.

Die erfindungsgemässen Dimeren bzw. niederen Oligomeren mit n $\leqslant$ 1 lassen sich in ihrer niedermolekularen bzw. niederoligomeren Form mit Vorteil zum Aufbau von Polyurethansystemen benutzen. Sie lassen sich insbesondere zur Herstellung von Einkomponentenreaktivsystemen entsprechend DE-OS Nr. 3131780 benutzen.

### *Herstellungsbeispiele*

*Beispiel 1* (Vergleichsversuch)

a) *Herstellung von dimerem Diphenylmethandiisocyanat nach bekannten Verfahrensweisen*

Zu einer Lösung von 1000 g (4,0 mol) Diphenylmethan-4,4'-diisocyanat (MDI) in 2000 g Toluol werden bei Raumtemperatur 1,5 g Tributylphosphin zugesetzt. Nach kurzer Zeit scheidet sich das dimere MDI aus der Lösung aus, wobei die Temperatur allmählich auf 30 bis 35°C ansteigt. Der Reaktionsansatz wird weitere 4 h gerührt, der Dimerisierungskatalysator durch Zugabe von 1,5 g Toluolsulfonsäuremethylester gestoppt und das feste MDI-Dimer abgesaugt. Nach ca. 5stündigem Trocknen bei 70 bis 80°C erhält man ca. 800 g MDI-Dimer in Form eines in fast allen Lösungsmitteln unlöslichen, feinen Pulvers. Der Zersetzungspunkt liegt oberhalb von 250°C.

Da die übliche Titration auf freie NCO-Gruppen in Aceton oder Dimethylformamid mittels Dibutylamin, insbesondere bei hochmolekularem MDI-Dimer (unlösliche Rückstände, Aufspaltung des Uretdionringes) zu sehr ungenauen Werten führt, wurde der freie NCO-Gehalt durch Reaktion mit Dibenzylamin als sterisch gehindertem Amin bestimmt, wobei die aufgenommene Aminmenge gravimetrisch ermittelt wurde.

Dazu wird eine Suspension von 10 g MDI-Dimer in 150 ml Toluol und 15 g Dibenzylamin (Überschuss) 30 min auf 50°C erwärmt, danach das Festprodukt isoliert und die Gewichtszunahme bestimmt – überraschenderweise lassen sich die NCO-Gruppen des di- und oligomeren MDI-Uretdiondiisocyanates auch mit dem sterisch gehinderten Diamin 2,4-Diamino-3,5-diethyltoluol mit hinreichender Genauigkeit bestimmen. Es entstehen hierbei $NH_2$-terminierte Polyharnstoffe mit noch intaktem Uretdionring, wobei bei niedermolekularem MDI-Dimer die Aminaufnahme (Reaktion mit freien NCO-Gruppen) am höchsten ist. Bei einem definierten Additionsprodukt von 2 mol sterisch gehindertem Amin an 1 mol des MDI-Dimeren ergeben sich bei verschiedenen Polyadditionsgraden vom MDI-Dimer (= n) verschiedene Aminaufnahmen. Aus der Dibenzylaminaufnahme von MDI-Dimer lässt sich der NCO-Gehalt und der Wert von n, bzw. das Molgewicht, errechnen (s. Tabelle).

*Tabelle*

| n | Molekulargewicht | Aminaufnahme g/10 g MDI-Dimer | NCO (berechnet) (%) |
|---|---|---|---|
| 0 | 500 | 7,88 | 16,8 |
| 1 | 750 | 5,26 | 11,2 |
| 2 | 1000 | 3,94 | 8,4 |
| 3 | 1250 | 3,15 | 6,7 |
| 4 | 1500 | 2,62 | 5,6 |

Das nach obiger Darstellungsmethode hergestellte MDI-Dimer zeigt eine Aminaufnahme von 3,0 g pro 10 g MDI-Dimer, entsprechend NCO berechnet = 6,4%. Ein solches sogenanntes MDI-Dimeres ist für die vorgesehenen Polyurethanreaktionen nicht geeignet (n > 3).

b) *Herstellung von erfindungsgemäss geeigneten Dimeren, bzw. nur niedere Oligomerwerte aufweisenden Diphenylmethanuretdiondiisocyanaten*

Wird wie in Beispiel 1a die Dimerisierung durchgeführt, das Rohprodukt nach dem Absaugen jedoch mit Petrolether gewaschen und ausschliesslich bei niedrigen Temperaturen bis etwa 30°C im Vakuum getrocknet, so erhält man ein niedermolekulares Uretdiondiisocyanat mit einer Aminaufnahme von 7,2 g (entsprechend einem berechneten NCO-Wert von 15,3%, d.h. einem Wert von n von 0,2). Im Gegensatz zu dem hocholigomerisierten MDI-Derivat nach Beispiel 1a ist dieses Diisocyanat (weitgehend Dimeres) zur Herstellung von hochelastischen Polyurethanen geeignet.

c) *Vergleich verschiedener Lösungsmittel bezüglich der Dimerisierungsreaktion*

Zu einer Lösung 100 g Diphenylmethan-4,4'-diisocyanat in jeweils 100 g der unten in der Tabelle aufgeführten Lösungsmittel werden 0,25 g Tributylphosphin zugesetzt. Bereits nach kurzer Zeit scheidet sich das Dimere in Form eines weissen Niederschlages aus. Es wird weitere 2 h bei Raumtemperatur gerührt und danach der Dimerisierungskatalysator durch 0,25 g Toluolsulfonsäuremethylester gestoppt. Das MDI-Dimer wird abgesaugt und nach Waschen mit Petrolether ohne Erhitzen bei Raumtemperatur im Vakuum getrocknet.

In der folgenden Tabelle werden die Ausbeuten (Prozent der Theorie), die aufgenommene Menge an sterisch gehindertem Amin pro 10 g Dimer (vgl. Beispiel 1a) und der daraus berechnete NCO-Gehalt des Dimeren, bzw. der Oligomeren, n angegeben.

| Lösungsmittel | MDI-Dimer-ausbeute (% d.Th.) | Aminaufnahme g/10 g MDI-Dimer | Mittleres Molgewicht | NCO (berechnet) (%) | n (berechnet) |
|---|---|---|---|---|---|
| 1. Tetrahydrofuran | 55 | 3,3 | 1175 | 7,1 | 2,7 |
| 2. Dioxan | 50 | 1,7 | | 3,54 | 7,5 |
| 3. Chloroform | 90 | 3,0 | | 6,4 | 3,3 |
| 4. Methylenchlorid | 88 | 3.0 | | 6,4 | 3,3 |
| 5. Dichlorethan-1,2 | 92 | 4,4 | 900 | 9,4 | 1,6 |
| 6. Toluol | 79 | 7,20 | 550 | 15,3 | 0,20 |
| 7. Essigester | 62 | 7,49 | 525 | 16,0 | 0,10 |
| 8. Aceton | 75 | 7,44 | 530 | 15,85 | 0,12 |
| 9. Chlorbenzol | 75 | 7,44 | 530 | 15,85 | 0,12 |
| 10. Bensoesäureethylester | 80 | 7,20 | 530 | 15,3 | 0,2 |
| 11. Phthalsäuredioctylester | 72 | 6,70 | 588 | 14,3 | 0,35 |

In stark polarem Lösungsmittel werden also unter diesen Reaktionsbedingungen durchweg hochmolekulare Diphenylmethanuretdione erhalten (vgl. 1 bis 4 in der Tabelle), die – vgl. DE-OS Nr. 3131780 – für bestimmte Polyurethanreaktionen wenig oder nicht geeignet sind. Produkte nach 5 ergeben nur relativ weiche Elastomere, sind jedoch noch brauchbar.

Die unter 6 bis 10 charakterisierten Produkte sind niedermolekulare Oligomere ($n \leqslant 1$) und somit für weitere Polyurethanreaktionen geeignet. Werden diese MDI-Uretdione jedoch einige Zeit bei 100°C getempert, so erfolgt weitere Umsetzung an den noch freien NCO-Gruppen zu hochmolekularem MDI-Dimer (vgl. Beispiel 3).

*Beispiel 2* (erfindungsgemäss)

Zu einer Lösung von 100 g MDI in 100 g Toluol werden jeweils 100 g des untenstehenden unpolaren Lösungsmittels zugesetzt. Zu der homogenen Lösung wurden nunmehr 0,25 g Tributylphosphin zugerührt. Nach kurzer Zeit scheidet sich das niedermolekulare Diphenylmethanuretdiondiisocyanat (MDI-Dimer) in Form eines weissen Niederschlages ab. Es wird noch 2 h bei Raumtemperatur weitergerührt. Danach wird abgesaugt und das MDI-Dimere nach Waschen mit Petrolether im Vakuum bei Raumtempermertur unter Feuchtigkeitsschluss getrocknet. In nachfolgender Tabelle werden die Ausbeute (Prozent der Theorie), die aufgenommene Menge an Dibenzylamin pro 10 g MDI-Dimer und der daraus berechnete NCO-Gehalt des MDI-Dimers angegeben.

| unpolares Lösungsmittel | MDI-Dimer-ausbeute (% d.Th.) | Aminaufnahme/10 g MDI-Dimer | NCO (berechnet) (%) | Molekulargewicht | n (berechnet) |
|---|---|---|---|---|---|
| a) Ligroin | 92 | 7,52 | 16,1 | 522 | 0,09 |
| b) Waschbenzin | 95 | 7,2 | 15,3 | 549 | 0,2 |
| c) Leichtbenzin | 90 | 7,89 | 16,7 | 503 | 0,012 |
| d) Cyclohexan | 96 | 7,60 | 16,3 | 515 | 0,06 |
| e) Hexan | 90 | 7,75 | 16,5 | 509 | 0,04 |
| f) Petrolether | 97 | 7,88 | 16,8 | 500 | 0,0 |

Bei $n = 0$ beträgt die berechnete Aminaufnahme 7,88 g/10 g MDI-Dimer.

Nach dem Verfahren werden also sehr niederoligomere Uretdione des Diphenylmethan-4,4'-uretdiondiisocyanats in einer ausgezeichneten Ausbeute erhalten.

Ähnlich günstige Werte erhält man, wenn man statt Toluol Essigester als mässig polares Medium mit gleichen Teilen unpolarem Lösungsmittel (Petrolether) verwendet.

*Beispiel 3*

*Einfluss der Temperungsbedingungen auf niedermolekulares MDI-Dimer von Beispiel 2*

Wird das gemäss Beispiel 2 aus Ligroin erhaltene MDI-Dimer bei 100°C verschieden lange getempert, so erhält man nach den in untenstehender Tabelle angeführten Zeiten folgende höhermolekularen Produkte:

| | Zeit (min) | Aminaufnahme g/10 g MDI-Dimer | NCO (berechnet) (%) | n (berechnet) | mittleres Molgewicht |
|---|---|---|---|---|---|
| 1. | 0 | 7,2 | 15,3 | 0,2 | 550 |
| 2. | 15 | 6,42 | 13,4 | 0,51 | 627 |
| 3. | 30 | 6,75 | 12,1 | 0,77 | 692 |
| 4. | 45 | 5,2 | 11,1 | 1,03 | 757 |
| 5. | 60 | 3,65 | 7,8 | 2,3 | 1075 |
| 6. | 120 | 0,22 | 0,5 | 60 | hochmolekular (~17000) |

Bei Lagerung bei 25°C bleibt dagegen das Ausgangsprodukt noch drei Monate praktisch unverändert (n = 0,12).

## Patentansprüche

1. Verfahren zur Herstellung von niedermolekularen 4,4'-Diphenylmethanuretdiondiisocyanaten der Formel

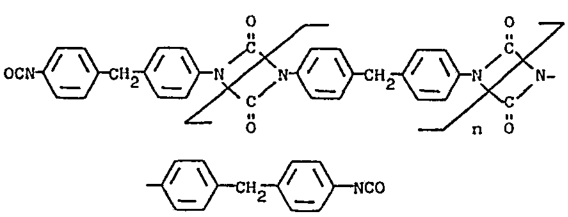

wobei n 0 bis 1, vorzugsweise 0 bis 0,5, bedeutet, durch Dimerisierung von Diphenylmethan-4,4'-diisocyanat in NCO-inerten Lösungsmitteln in Gegenwart von Dimerisierungskatalysatoren und gegebenenfalls Abstoppern und Einhaltung einer Temperatur bei der Dimerisierungsreaktion und Aufarbeitung von ≤50°C, dadurch gekennzeichnet, dass man die Dimerisierung in Gegenwart von Dimerisierungskatalysatoren in einem Gemisch aus unpolaren Lösungsmitteln und mässig polaren Lösungsmitteln auf der Basis von aromatischen Kohlenwasserstoffen, gegebenenfalls ihrer chlorierten Derivate, von Carbonsäureestern, von Ketonen und von Ethern, mit Dielektrizitätskonstanten von 2,24 bis 10, im Falle von Ketonen bis 22, durchführt, wobei die Löslichkeit des Diphenylmethan-4,4'-diisocyanats in den unpolaren Lösungsmitteln 5 bis 25 Gew.-% beträgt.

2. Verfahren nach Anspruch 1, dadurch gekennzeichet, dass man ein Gemisch aus unpolaren Lösungsmitteln und mässig polaren Lösungsmitteln im Mengenverhältnis 1:2 bis 2:1 verwendet, wobei die unpolaren Lösungsmittel aliphatische und/oder cycloaliphatische Kohlenwasserstoffe und die mässig polaren Lösungsmittel aromatische Kohlenwasserstoffe, ihre chlorierten Derivate, Carbonsäureester, Ketone und Ether darstellen.

3. Verfahren nach den Ansprüchen 1 und 2, dadurch gekennzeichnet, dass die Löslichkeit des Diphenylmethan-4,4'-diisocyanats in den unpolaren Lösungsmitteln 5 bis 15 Gew.-% bei Raumtemperatur beträgt und dass die Dielektizitätskonstante der unpolaren Lösungsmittel unter 2,10 liegt, und als mässig polare Lösungsmittel Benzol, Toluol, Xylol, Chlorbenzol, Essigsäuremethylester, Essigsäureethylester, Propionsäurediethylester, Ameisensäurediethylester, Phthalsäurediethylester, Phthalsäuredioctylester, Aceton Methylethylketon, Cyclohexanon, Isophoron, Diethylether, Tetrahydrofuran und Tetrachlorkohlenstoff verwendet werden.

4. Verfahren nach den Ansprüchen 1 bis 3, dadurch gekennzeichnet, dass als unpolare Lösungsmittel Petrolether und Benzin und als mässig polares Lösungsmittel Toluol verwendet wird.

5. Verfahren nach den Ansprüchen 1 bis 4, dadurch gekennzeichnet, dass man während der Dimerisierungsreaktion und bei der Aufarbeitung der Uretdiondiisocyanate Temperaturen unter 30°C einhält.

6. Verfahren nach den Ansprüchen 1 bis 5, dadurch gekennzeichnet, dass man 0,1 bis 1 Gew.-% an Trialkylphosphinen als Katalysator verwendet.

7. Verfahren nach den Ansprüchen 1 bis 6, dadurch gekennzeichnet, dass 1 bis 2 Gew.-Teile des unpolaren Lösungsmittels mit einem Teil Diphenylmethan-4,4'-diisocyanat bei Raumtemperatur bis zur Sättigung gerührt werden und anschliessend gerade so viel eines mässig polaren Lösungsmittels zugesetzt wird, bis eine homogene Lösung entsteht.

8. Verfahren nach den Ansprüchen 1 bis 7, dadurch gekennzeichnet, dass als Dimerisierungskatalysator Tributylphosphin verwendet wird.

## Revendications

1. Procédé de préparation de 4,4'-diphénylmé-

thane-uretdionediisocyanates de faible poids moléculaire répondant à la formule:

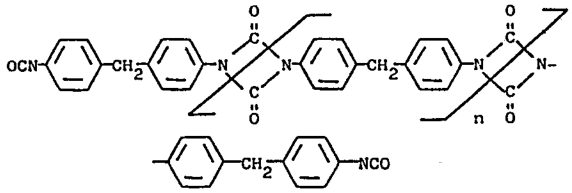

où n représente un nombre de 0 à 1, de préférence de 0 à 0,5, par dimérisation de diphénylméthane-4,4'-diisocyanate dans des solvants inertes vis-à-vis des groupes NCO, en présence de catalyseurs de dimérisation et éventuellement d'agent d'arrêt, et en maintenant une température de ⩽50°C lors de la réaction de dimérisation et du traitement, caractérisé en ce qu'on effectue la dimérisation en présence de catalyseurs de dimérisation dans un mélange de solvants non polaires et de solvants modérément polaires à base d'hydrocarbures aromatiques, éventuellement de leurs dérivés chlorés, d'esters d'acides carboxyliques, de cétones et d'éthers, avec des constantes diélectriques de 2,24 à 10 et allant jusqu'à 22 dans le cas des cétones, la solubilité du diphénylméthane-4,4'-diisocyanate dans les solvants non polaires étant de 5 à 25% en poids.

2. Procédé suivant la revendication 1, caractérisé en ce qu'on utilise un mélange de solvants non polaires et de solvants modérément polaires dans un rapport quantitatif de 1:2 à 2:1, les solvants non polaires étant des hydrocarbures aliphatiques et/ou cycloaliphatiques, tandis que les solvants modérément polaires sont des hydrocarbures aromatiques, leurs dérivés chlorés, des esters d'acides carboxyliques, des cétones et des éthers.

3. Procédé suivant les revendications 1 et 2, caractérisé en ce que la solubilité du diphénylméthane-4,4'-diisocyanate dans les solvants non polaires est de 5 à 15% en poids à la température ambiante, la constance diélectrique des solvants non polaires est inférieure à 2,10 et, comme solvants modérément polaires, on utilise le benzène, le toluène, le xylène, le chlorobenzène, l'ester méthylique d'acide acétique, l'ester éthylique d'acide acétique, l'ester diéthylique d'acide propionique, l'ester diéthylique d'acide formique, l'ester diéthylique d'acide phtalique, l'ester dioctylique d'acide phtalique, l'acétone, la méthyléthylcétone, la cyclohexanone, l'isophorone, l'éther diéthylique, le tétrahydrofuranne et le tétrachlorure de carbone.

4. Procédé suivant les revendications 1 à 3, caractérisé en ce que, comme solvants non polaires, on utilise l'éther de pétrole et l'essence et, comme solvant modérément polaire, on utilise le toluène.

5. Procédé suivant les revendications 1 à 4, caractérisé en ce que, pendant la réaction de dimérisation et lors du traitement des uretdionediisocyanates, on maintient des températures inférieures à 30°C.

6. Procédé suivant les revendications 1 à 5, caractérisé en ce que, comme catalyseur, on utilise 0,1 à 1% en poids de trialkylphophines.

7. Procédé suivant les revendications 1 à 6, caractérisé en ce qu'on agite 1 à 2 parties en poids du solvant non polaire avec 1 partie de diphénylméthane-4,4'-diisocyanate à la température ambiante jusqu'à saturation, puis on ajoute un solvant modérément polaire précisément jusqu'à ce qu'il se forme une solution homogène.

8. Procédé suivant les revendications 1 à 7, caractérisé en ce que, comme catalyseur de dimérisation, on utilise la tributylphosphine.

## Claims

1. A process for the production of low molecular weight 4,4'-diphenylmethane-uretdione-diisocyanates corresponding to the following formula

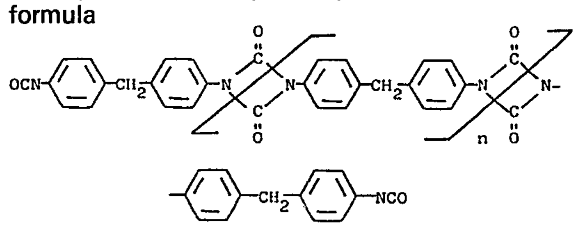

wherein n is from 0 to 1, preferably from 0 to 0.5, by the dimerisation of diphenylmethane-4,4'-diisocyanate in NCO-inert solvents, in the presence of dimerisation catalysts and optionally stoppers, and maintaining a temperature during the dimerisation reaction and the work-up of ⩽50°C, characterised in that the dimerisation is carried out in the presence of dimerisation catalysts in a mixture of non-polar and moderately polar solvents, based on aromatic hydrocarbons, optionally the chlorinated derivatives thereof, carboxylic acid esters, ketones and ethers having a dielectric constant of from 2.24 to 10, and up to 22 in the case of ketones, the solubility of the diphenylmethane-4,4'-diisocyanate in the non-polar solvents being from 5 to 25% by weight.

2. A process according to Claim 1, characterised in that a mixture of non-polar solvents and moderately polar solvents is used in a quantity ratio of from 1:2 to 2:1, the non-polar solvents being aliphatic and/or cycloaliphatic hydrocarbons, the chlorinated derivatives thereof, carboxylic acid esters, ketones and ethers.

3. A process according to Claims 1 and 2, characterised in that the solubility of the diphenylmethane-4,4'-diisocyanate in the non-polar solvents is from 5 to 15% by weight and the dielectric constant of the non-polar solvents is below 2.10, and benzene, toluene, xylene, chlorobenzene acetic acid methylesters, acetic acid ethylesters, propionic acid diethylesters, formic acid diethylesters, phthalic acid diethylesters, phthalic acid dioctylesters, acetone, methylethylketone, cyclohexanone, isophorone, diethylether, tetrahydrofuran and tetracarbon choride are used as moderately polar solvents.

4. A process according to Claims 1 to 3, characterised in that petroleum ether and petrol are used as non-polar solvents and toluene is used as a moderately polar solvent.

5. A process according to Claims 1 to 4,

characterised in that a temperature of below 30°C is maintained during the dimerisation reaction and the work-up of the uretdione-diisocyanates.

6. A process according to Claims 1 to 5, characterised in that from 0.1 to 1% by weight of trialkyl phosphines are used as the catalyst.

7. A process according to Claims 1 to 6, characterised in that from 1 to 2 parts by weight of the non-polar solvent are stirred with 1 part of diphenylmethane-4,4'-diisocyanate at room temperature up to saturation, and then just enough of a moderately polar solvent is added to produce a homogeneous solution.

8. A process according to Claims 1 to 7, characterised in that tributyl phosphine is used as the dimerisation catalyst.